# EUROPEAN PATENT APPLICATION

(11) **EP 3 845 120 A1**
(43) Date of publication of application: **07.07.2021**
(21) Application number: 20152739.7
(22) Date of filing: 20.01.2020
(51) Int. Cl.: A61B 5/00

(54) **PHYSIOLOGICAL WAVEFORM SUMMARIZING SYSTEM AND METHOD**

(30) Priority: 31.12.2019 US 201962955890 P
(71) Applicant: Koninklijke Philips N.V., 5656 AG Eindhoven (NL)
(72) Inventor: Firoozabadi, Reza, 5656 AE Eindhoven (NL); Babaeizadeh, Saeed, 5656 AE Eindhoven (NL)
(74) Representative: Steenbeek, Leonardus Johannes

(57) **Abstract**

A physiological waveform summarizing system encompasses a physiological waveform (CPW) interface (20), and a physiological waveform summarizing monitor (30). In operation, the monitor (30) extracts a set of dominant physiological templates (41) from a physiological waveform (CPW) communicated by the interface (20) to the monitor (30). The set of dominant physiological templates (41) represent a dominating major physiological rhythm (DMPR) of the physiological waveform (CPW) temporally spanning over consecutive intervals of the physiological waveform (CPW), and each dominant physiological template (41) is derived from a different interval of the consecutive intervals of the physiological waveform (CPW). The monitor (30) may extract one or more secondary physiological templates (42) representative of secondary major physiological rhythm(s) (SMPR) present in the physiological waveform (CPW), and provide a diagnostic major physiological rhythm log of the physiological waveform (CPW) including a diagnostic plotting of the dominant physiological templates (41) and any secondary physiological template(s) (42).

## Description

### FIELD OF THE INVENTION

The present disclosure generally relates to a diagnostic monitoring and interpretation of physiological waveforms (e.g., electrocardiograms (ECG), photoplethysmogram (PPG) and capnogram (CO₂)). The present disclosure specifically relates a summarizing of a physiological waveform to facilitate a more accurate and reliable diagnostic monitoring and interpretation of the physiological waveform.

### BACKGROUND OF THE INVENTION

Many physiological waveforms (e.g., electrocardiograms (ECG), photoplethysmograms (PPG) and capnograms (CO₂)) display a repeated pattern of limited number of shapes. For example, in a resting healthy person, a standard recording of ECG could simply look like one particular shape for one heart beat is repeated over and over. Of course, even in such case there really are minor differences between these similar-looking "beats" due to both physiological and environmental reasons. These changes may be more pronounced in unhealthy subjects and/or when the environmental conditions change. Therefore, monitoring the changes in repeating pattern is widely used to screen or diagnose a disease.

For example, a stress test (sometimes called a treadmill test or exercise test) gradually increases the workload on the subject's heart in order to find out how well the heart handles work. In the presence of obstructive coronary artery disease (CAD), the morphology of ECG beats changes. More precisely, usually the shape of ST segment in the ECG beat changes, through either depression or elevation. Such morphology changes often suggest presence of CAD and warrants further management.

Physicians and the expert reviewers of the physiological waveforms are often given long recordings to annotate and extract the abnormalities and events by looking for the changes in repeating patterns and standard parameters determined by measurements. This procedure is performed either manually or by the aid of computer applications and is usually difficult, time-consuming and error-prone especially in presence of noise and artifact.

ECG, as a typical example of a physiological waveform reviewed by experts, is the main tool in identification of the life-threatening arrhythmias, and detection of the cardiac diseases by measuring the standard parameters from beat fiducial points, such as, for example, ST elevation, QT duration, and QRS width, and inspection of the presence of normal ECG beat components such as P-wave and normal T-wave.

In a long record of a physiological waveform, it would be challenging and time-consuming to detect the gradual and major changes to the repeating signal pattern (i.e. pulses). Furthermore, keeping track of all changes and measurements could be impacted by human error factors. Additionally, the noise and artifact on the signal will make it difficult to make valid and reliable measurements of the pattern.

Moreover, a failure in detection and reliable measurement of each of these parameters in an ECG record reviewed by experts may result in drastic and irreversible errors with potentially detrimental consequences for the patient undergoing the diagnosis.

### SUMMARY OF THE INVENTION

The invention is defined by the independent claims. The dependent claims define advantageous embodiments. The present disclosure describes physiological waveform summarizing that is applicable to numerous and various diagnostic monitoring and interpretation applications of physiological waveforms (e.g., ECG waveform, PPG waveform, CO₂ waveform, etc.).

The physiological waveform summarizing of the present disclosure identifies one or more major physiological templates for each interval of consecutive intervals (overlapping or non-overlapping) of a physiological waveform to thereby exclude minor physiological templates, which may contain noise and/or artefacts, from the physiological waveform.

One major physiological template is a dominant physiological template constituting a dominating major physiological rhythm of the physiological waveform within an interval of the physiological waveform whereby any deviation in the morphology of the dominating major physiological rhythm may be an indication of a predominant abnormality in the physiological waveform, and further whereby a diagnostic analysis of physiological parameters of the dominating major physiological rhythm will be more accurate and reliable than diagnostic analysis of physiological parameters of the physiological waveform.

A second major physiological template is a secondary physiological template constituting a presence of a secondary major physiological rhythm within an interval of the physiological waveform. A secondary physiological template is usually a sign of an underlying abnormality in the physiological waveform. For example, in an ECG rhythm, a secondary template may be indicative of arrhythmias such as Premature Atrial Complex (PAC), Premature Ventricular Complex (PVC - bigeminy or trigeminy rhythms), Atrial Fibrillation (AFib), Atrial Flutter (AFlutter), and Ventricular Tachycardia (VT).

The present disclosure may be embodied as:
(1) a physiological waveform summarizing monitor of the present disclosure;
(2) a physiological waveform summarizing system incorporating a physiological waveform summarizing monitor of the present disclosure; and
(3) a physiological waveform summarizing method utilizing a physiological waveform summarizing monitor of the present disclosure.

Various embodiments of a physiological waveform summarizing monitor of the present disclosure encompass a physiological waveform summarizing recorder and a physiological waveform summarizing analyzer for summarizing a physiological waveform having a dominating major physiological rhythm and possibly one or more secondary major physiological rhythms.

In operation, the physiological waveform summarizing recorder receives a physiological waveform in real-time or as a recording. The recorder processes the physiological waveform to extract a set of dominant physiological templates from the physiological waveform. The set of dominant physiological templates represent the dominating major physiological rhythm of the physiological waveform temporally spanning over consecutive intervals of the physiological waveform, and each dominant physiological template is derived from a different interval of the consecutive intervals of the physiological waveform.

The recorder may also extract one or more secondary physiological template from the physiological waveform. The secondary physiological template(s) represent(s) secondary major physiological rhythm(s) of the physiological waveform.

In operation, the physiological waveform summarizing analyzer provides a major physiological rhythm log of the physiological waveform including a diagnostic plotting of one or more of extracted dominant physiological templates, and optionally one or more of the extracted secondary physiological templates.

Various embodiments of a physiological waveform summarizing systems of the present disclosure encompass a physiological waveform interface in addition to a physiological waveform summarizing monitor of the present disclosure. The physiological waveform interface employs machines, devices, appliances, equipment, tools, accessories, etc. as known in the art of the present disclosure and hereinafter conceived for interfacing with a monitored subject to acquire and communicate the physiological waveform to the monitor. Examples of a physiological waveform interface include, but are not limited to, an ECG pads/ECG lead system based interface for acquiring and communicating an electrocardiogram (ECG) waveform to the monitor, a pulse oximeter based interface for acquiring and communicating a photoplethysmogram (PPG) waveform to the monitor and a CO₂ sensor based interface for acquiring and communicating a capnogram (CO₂) waveform to the monitor.

Various embodiments of a physiological waveform summarizing method of the present disclosure encompass a summary of a physiological waveform having a dominating major physiological rhythm and possibly one or more secondary major physiological rhythms.

In operation, the physiological waveform summarizing recorder receives a physiological waveform in real-time or as a recording. The recorder processes the physiological waveform to extract dominant physiological templates from the physiological waveform. The dominant physiological templates represent the dominating major physiological rhythm of the physiological waveform temporally spanning over consecutive intervals (overlapping or non-overlapping) of the physiological waveform, and each dominant physiological template is derived from a different interval of the consecutive intervals of the physiological waveform.

The recorder may also extract one or more secondary physiological template from the physiological waveform if secondary major physiological rhythm(s) are present in the physiological waveform.

In operation, the physiological waveform summarizing analyzer provides a major physiological rhythm log of the physiological waveform including a diagnostic plotting of one or more extracted dominant physiological templates, and one or more of any extracted secondary physiological template(s).

For purposes of the description and claims of the present disclosure:
(1) terms of the art including, but not limited to, "physiology (and tenses thereof)", "waveform", "interval", "interface (and tenses thereof)", "monitor (and tenses thereof)" and "log" are to be understood as known in the art of the present disclosure and as exemplary described in the present disclosure;
(2) the term "monitored subject" broadly encompasses a human or an animal subject to a monitoring and an interpretation, supervised or unsupervised, of a physiological status of the human or the animal as known in the art of the present disclosure and hereinafter conceived;
(3) the term "physiological waveform" broadly encompasses any type of periodic/cyclical signal indicative of a physiological status of a monitored subject, as known in the art of the present disclosure and hereinafter conceived, having a designated normal temporal shape susceptible to abnormal deviations thereof or a range of normal temporal shapes susceptible to abnormal deviations thereof. Examples of a physiological waveform include, but are not limited to, an electrocardiogram (ECG) waveform, a photoplethysmogram (PPG) waveform and a capnogram (CO₂) waveform;
(4) the term "dominating major physiological rhythm" broadly encompasses a major rhythm of the physiological waveform best characteristic of a physiological status of the monitored subject;
(5) the term "secondary major physiological rhythm" broadly encompasses a major rhythm of the physiological waveform underlying the dominating major physiological rhythm of the physiological waveform;
(6) the term "dominant physiological template" broadly encompasses a dominant major morphology within an interval of a physiological waveform in accordance with the present disclosure as exemplary described in the present disclosure. Examples of a dominant morphology include, but are not limited to, a mean morphology or a function thereof among the signal periods/cycles within an interval of a physiological waveform, a median morphology or a function thereof among the signal periods/cycles within an interval of a physiological waveform and a mode morphology or a function thereof among the signal periods/cycles within an interval of a physiological waveform.
(7) the term "secondary physiological template" broadly encompasses a presence of a secondary major morphology within an interval of a physiological waveform in accordance with the present disclosure as exemplary described in the present disclosure;
(8) the term "major physiological rhythm log" broadly encompasses a logging one or more of the dominant physiological templates for purpose of a diagnostic monitoring and interpretation of the physiological waveform in accordance with the present disclosure as exemplary described in the present disclosure. Examples of the logging of dominant physiological templates included, but are not limited to, a temporal logging of a subset or an entire set of dominant physiological templates or a deviant logging of a subset of dominant physiological templates having a major morphology deviation between the templates as exemplary described in the present disclosure;
(9) the term "physiological waveform summarizing monitor" broadly encompasses all structural configurations, as understood in the art of the present disclosure and as exemplary described in the present disclosure, of a monitor having main circuit board(s) and/or integrated circuit(s) for controlling an application of various principles of the present disclosure for summarizing a physiological waveform in accordance with the present disclosure;
(10) the term "application module" broadly encompasses an application incorporated within or accessible by a medical ventilator or a monitor consisting of an electronic circuit (e.g., electronic components and/or hardware) and/or an executable program (e.g., executable software stored on non-transitory computer readable medium(s) and/or firmware) for executing a specific application associated with a summarizing of a physiological waveform in accordance with the present disclosure; and
(11) the terms "signal", "data" and "command" broadly encompasses all forms of a detectable physical quantity or impulse (e.g., voltage, current, or magnetic field strength) as understood in the art of the present disclosure and as exemplary described in the present disclosure for transmitting information and/or instructions in support of applying various inventive principles of the present disclosure as subsequently described in the present disclosure. Signal/data/command communication various components of the present disclosure may involve any communication method as known in the art of the present disclosure including, but not limited to, signal / data / command transmission/reception over any type of wired or wireless datalink and a reading of signal/data/commands uploaded to a computer-usable/computer readable storage medium.

The foregoing embodiments and other embodiments of the present disclosure as well as various structures and advantages of the present disclosure will become further apparent from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments in accordance with the present disclosure with reference to the following figures wherein:
FIG. 1A illustrates an exemplary embodiment of a physiological waveform summarizing system;
FIG. 1B illustrates an exemplary embodiment of a flowchart representative of a physiological waveform summarizing method;
FIG. 2A illustrates an exemplary embodiment of an electrocardiogram (ECG) waveform summarizing system;
FIG. 2B illustrates an exemplary embodiment of a flowchart representative of an electrocardiogram (ECG) waveform summarizing method;
FIG. 3A illustrates an exemplary embodiment of a photoplethysmogram (PPG) waveform summarizing system;
FIG. 3B illustrates an exemplary embodiment of a flowchart representative of a photoplethysmogram (PPG) waveform summarizing method;
FIG. 4A illustrates an exemplary embodiment of a capnogram (CO2) waveform summarizing system;
FIG. 4B illustrates an exemplary embodiment of a flowchart representative of a capnogram (CO2) waveform summarizing method;
FIG. 5A illustrates an exemplary embodiment of a flowchart representative of a physiological template plotting/logging method;
FIG. 5B illustrates an exemplary embodiment of a flowchart representative of physiological template matching method;
FIG. 5C illustrates an exemplary embodiment of a flowchart representative of physiological template selection method;
FIGS. 6A-6C illustrate a first exemplary ECG waveform summarizing in accordance with the present disclosure;
FIGS. 7A-7C illustrate a second exemplary ECG waveform summarizing;
FIGS. 8A-8C illustrate a third exemplary ECG waveform summarizing; and
FIG. 9 illustrates an exemplary embodiment of a physiological waveform summarizing monitor.

### DETAILED DESCRIPTION OF EMBODIMENTS

The present disclosure is applicable to numerous and various diagnostic monitoring and interpretation applications of physiological waveforms (e.g., ECG waveform, PPG waveform, CO₂ waveform, etc.).

Dominant physiological templates are extracted from a physiological waveform. The dominant physiological templates represent a dominating major physiological rhythm of the physiological waveform, whereby a diagnostic analysis of physiological parameters of the dominant physiological templates will be more accurate and reliable than a diagnostic analysis of the dominating major physiological rhythm of the physiological waveform.

Optionally, secondary physiological templates are extracted from the physiological waveform. The secondary physiological templates represent a secondary major physiological rhythm of the physiological waveform, and whereby an analysis of the physiological parameters of the secondary physiological templates will be more accurate and reliable than an analysis of the secondary major physiological rhythm of the physiological waveform.

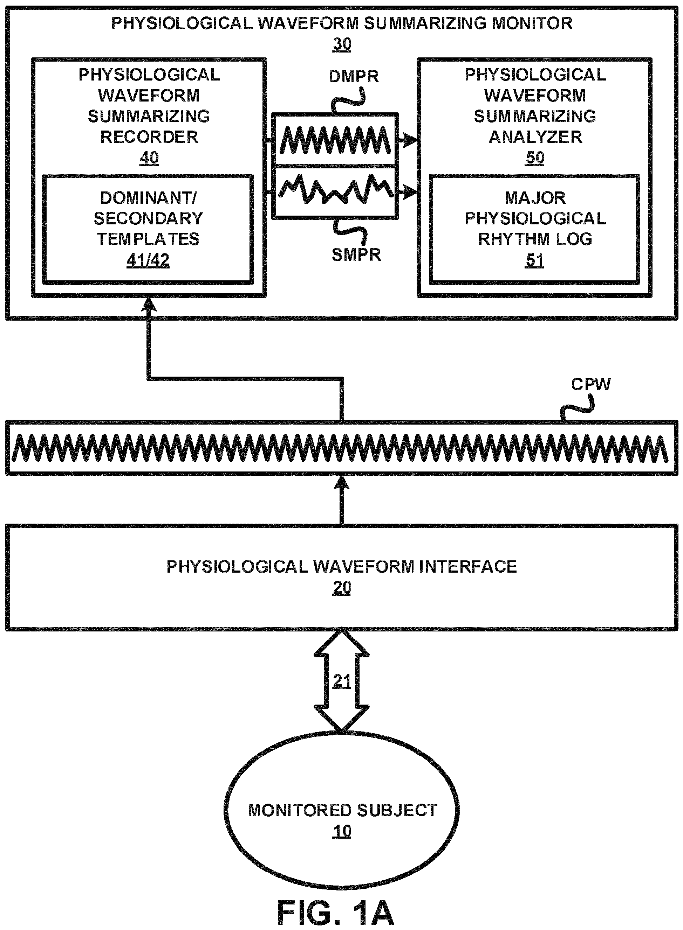

To facilitate an understanding of the present disclosure, the following description of FIGS. 1A-4B teaches exemplary embodiments of physiological waveform summarizing monitors, systems and methods in accordance with the present disclosure. From the description of FIGS. 1A-4B, those having ordinary skill in the art of the present disclosure will appreciate how to apply the present disclosure to make and use additional embodiments of physiological waveform summarizing monitors, systems and methods in accordance with the present disclosure.

Referring to FIG. 1A, a physiological waveform summarizing system of the present disclosure employs a physiological waveform interface 20 as known in the art and a physiological waveform summarizing monitor 30 of the present disclosure. In practice, interface 20 employs machines, devices, appliances, equipment, tools, accessories, etc. as known in the art of the present disclosure and hereinafter conceived for interfacing 21 with a monitored subject 10 to acquire and communicate a physiological waveform CPW to monitor 30.

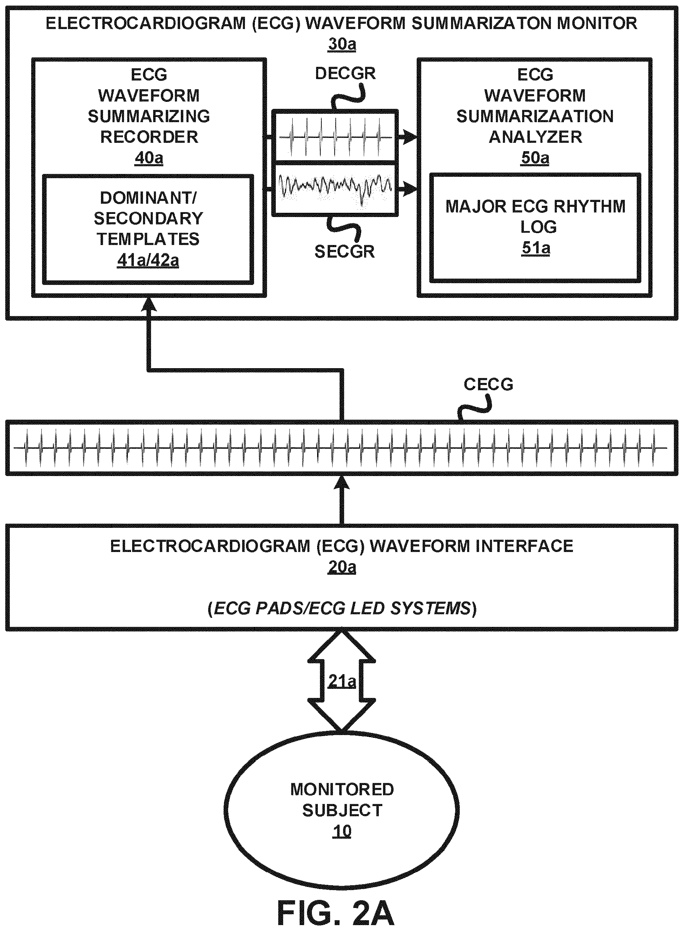

In one exemplary embodiment of interface 20 as shown in FIG. 2A, an electrocardiogram waveform interface 20a employs ECG pads or an ECG lead system and associated accessories as known in the art of the present disclosure and hereinafter conceived for interfacing 21a with monitored subject 10 to acquire and communicate an electrocardiogram waveform CECG to an electrocardiogram waveform summarizing monitor 30a.

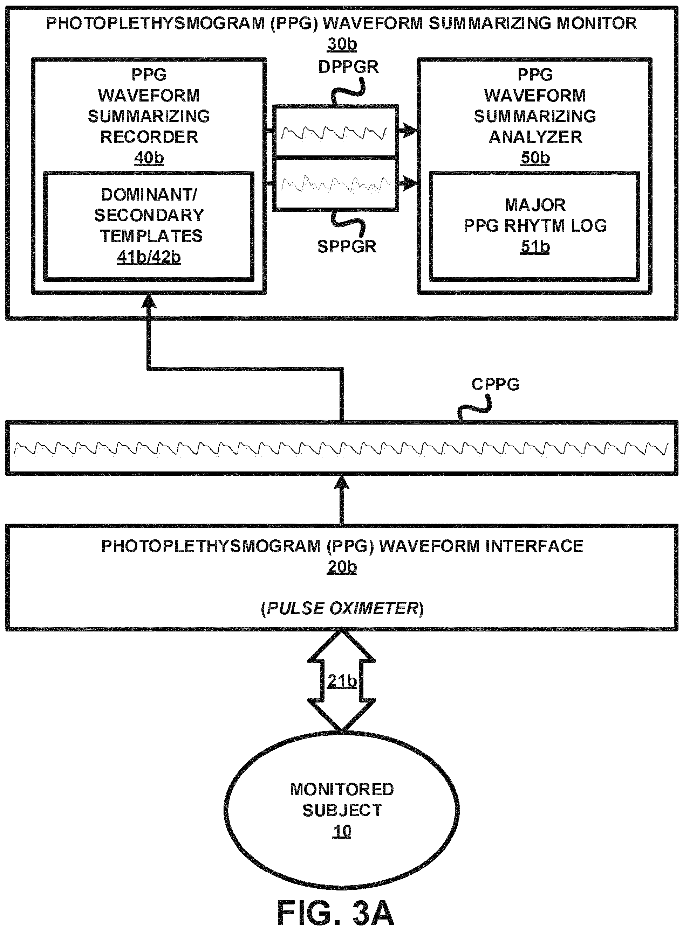

In a second exemplary embodiment of interface 20 as shown in FIG. 3A, a photoplethysmogram waveform interface 20b employs a pulse oximeter and associated accessories as known in the art of the present disclosure and hereinafter conceived for interfacing 21b with monitored subject 10 to acquire and communicate a photoplethysmogram waveform CPPG to a photoplethysmogram waveform summarizing monitor 30b.

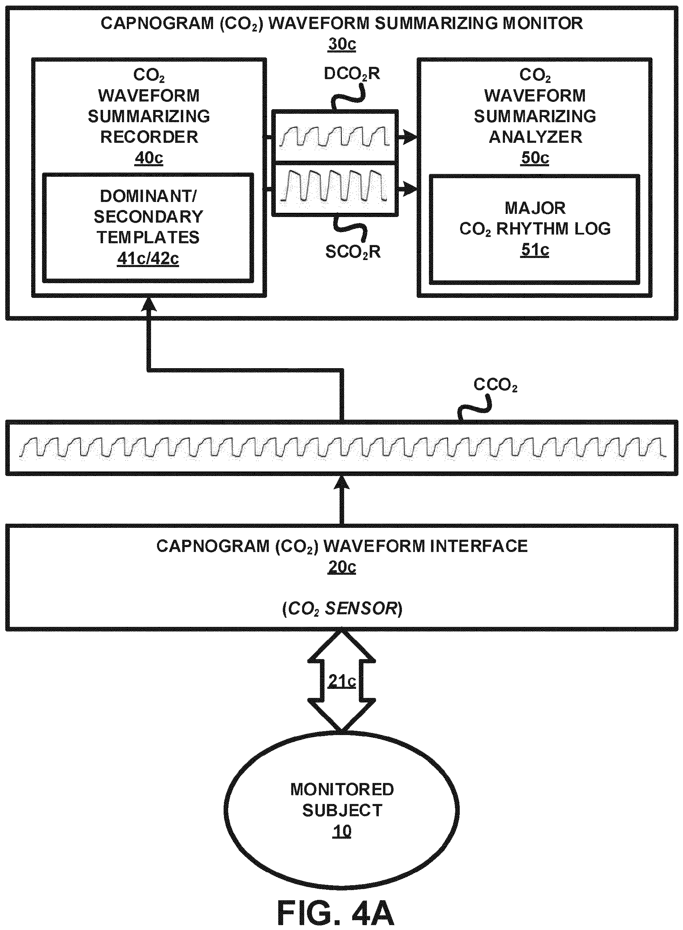

In a third exemplary embodiment of interface 20 as shown in FIG. 4A, a capnogram waveform interface 20c employs carbon dioxide (CO₂) sensors and associated accessories as known in the art of the present disclosure and hereinafter conceived for interfacing 21c with monitored subject 10 to acquire and communicate a capnogram waveform CCO₂ to a capnogram waveform summarizing monitor 30c.

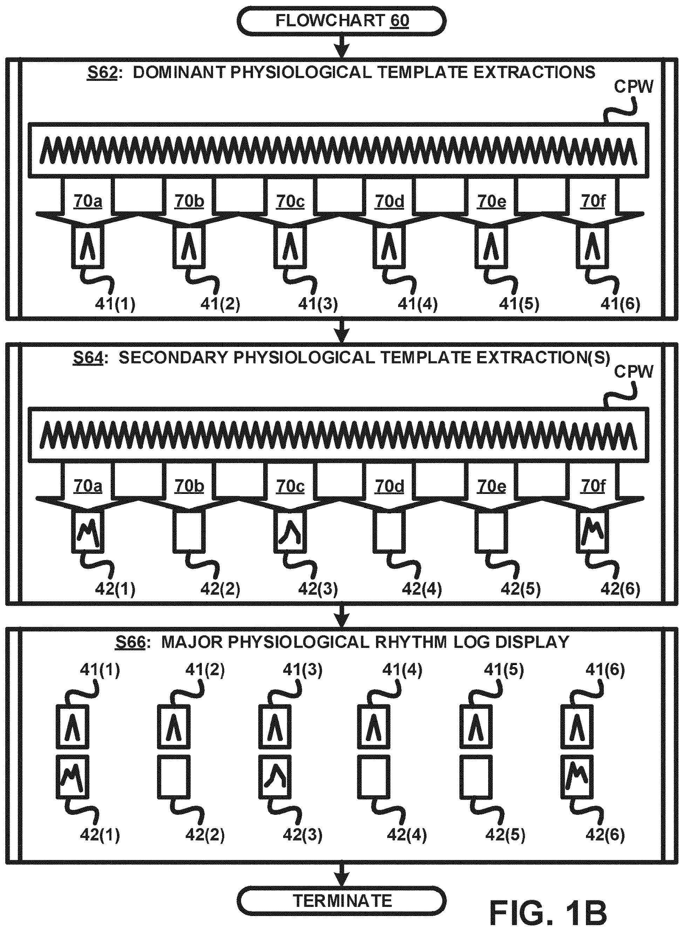

Referring back to FIG. 1A, monitor 30 employs a physiological waveform summarizing recorder 40 and a physiological waveform summarizing analyzer 50 for implementing a physiological waveform summarizing method of the present disclosure as represented by a flowchart 60 shown in FIG. 1B.

Recorder 40 records physiological waveform CPW as received from physiological waveform interface 20. Concurrently or subsequently to the recording of physiological waveform CPW, recorder 40 extracts dominant physiological templates 41 from physiological waveform CPW.

During a stage S62 of flowchart 60, recorder 40 identifies a dominant physiological template 41 for each interval of a plurality consecutive intervals (overlapping or non-overlapping) of physiological waveform CPW. For example, as shown in stage S62, physiological waveform CPW is segmented into six (6) intervals 70a-70f whereby a dominant morphology within each interval 70 of physiological waveform CPW is extracted and designated as a dominant physiological template 41.

A dominant morphology of an interval of physiological waveform CPW is the morphology best representative of a physiological status of the monitored subject during that interval of physiological waveform CPW for diagnostic monitoring and interpretation purposes.

Examples of a dominant morphology of an interval of physiological waveform CPW include, but are not limited to, a mean morphology or function thereof among the periods/cycles within the interval of physiological waveform CPW, a median morphology or function thereof among the periods/cycles of within the interval of physiological waveform CPW, and a mode morphology or function thereof among the periods/cycles of within the interval of physiological waveform CPW.

At the conclusion of stage S62, recorder 40 temporally aligns the dominant physiological templates 41 as a representation of a dominating major physiological rhythm DMPR of physiological waveform CPW, which may be communicated to analyzer 50 as a dominating major physiological rhythm as shown or as individual dominant physiological templates 41 in sequential order.

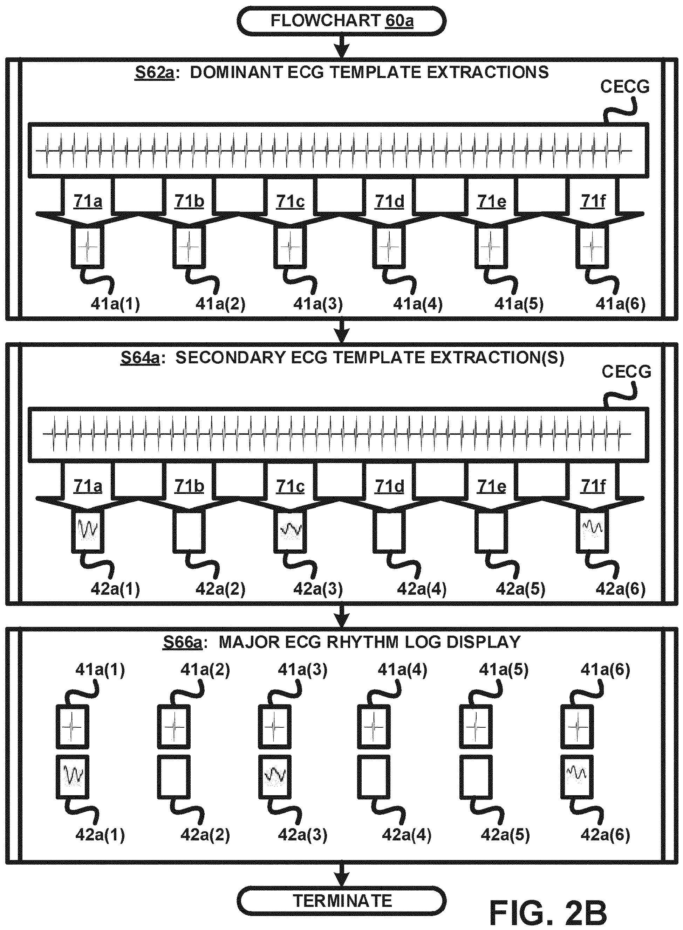

In one exemplary embodiment of monitor 30 as shown in FIG. 2A, an electrocardiogram waveform summarizing monitor 30a employs an electrocardiogram waveform summarizing recorder 40a and an electrocardiogram waveform summarizing analyzer 50a for implementing an electrocardiogram waveform summarizing method of the present disclosure as represented by a flowchart 60a shown in FIG. 2B.

Recorder 40a records electrocardiogram waveform CECG as received from electrocardiogram waveform interface 20a. Concurrently or subsequently to the recording of electrocardiogram waveform CECG, recorder 40a extracts dominant electrocardiogram templates 41a from electrocardiogram waveform CECG.

During a stage S62a of flowchart 60a, recorder 40a identifies a dominant electrocardiogram template 41a for each interval of consecutive intervals (overlapping or non-overlapping) of electrocardiogram waveform CECG. For example, as shown in stage S62a, electrocardiogram waveform CECG is segmented into six (6) intervals 71a-71f whereby a dominate morphology within each interval 71 of electrocardiogram waveform CECG is extracted and designated as a dominant electrocardiogram template 41a.

A dominant morphology of an interval of electrocardiogram waveform CECG is the morphology best representative of an electrocardiogram status of the monitored subject during that interval of electrocardiogram waveform CECG for diagnostic monitoring and interpretation purposes.

Examples of a dominant morphology of an interval of electrocardiogram waveform CECG include, but are not limited to, a mean morphology or function thereof among the cardiac cycles within the interval of electrocardiogram waveform CECG, a median morphology or function thereof among the cardiac cycles of within the interval of electrocardiogram waveform CECG, and a mode morphology or function thereof among the cardiac cycles of within the interval of electrocardiogram waveform CECG.

At the conclusion of stage S62a, recorder 40 temporally aligns the dominant electrocardiogram templates 41a as a representation of a dominant electrocardiogram rhythm DECGR of physiological waveform CPW, which may be communicated to analyzer 50a as dominant electrocardiogram waveform as shown or as individual dominant electrocardiogram templates 41a in sequential order.

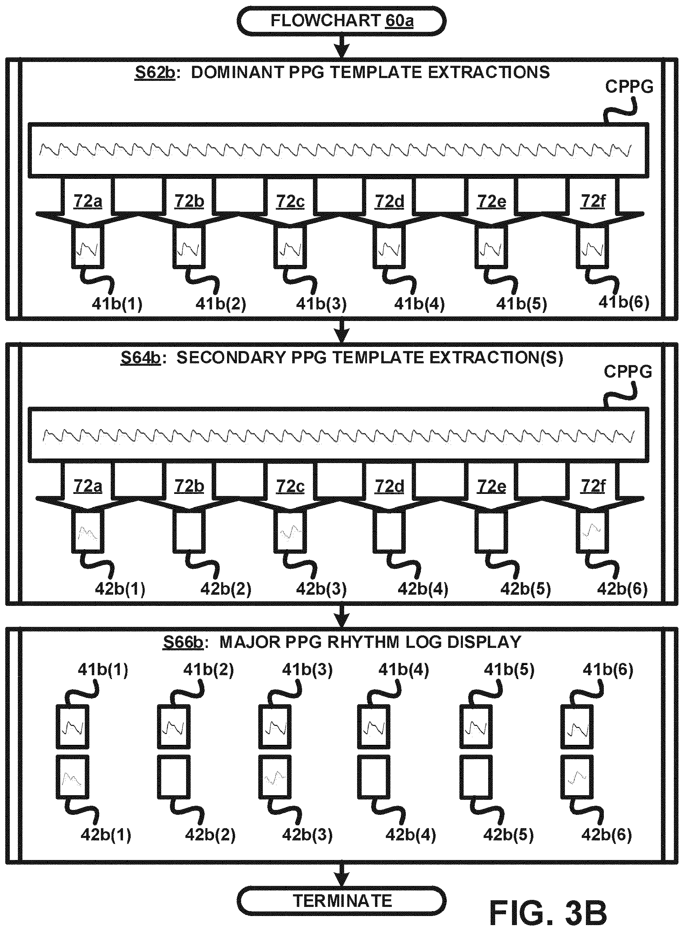

In a second exemplary embodiment of monitor 30 as shown in FIG. 3A, a photoplethysmogram waveform summarizing monitor 30b employs a photoplethysmogram waveform summarizing recorder 40b and a photoplethysmogram waveform summarizing analyzer 50b for implementing a photoplethysmogram waveform summarizing method of the present disclosure as represented by a flowchart 60b shown in FIG. 3B.

Recorder 40b records photoplethysmogram waveform CPPG as received from photoplethysmogram waveform interface 20a. Concurrently or subsequently to the recording of photoplethysmogram waveform CPPG, recorder 40b extracts dominant photoplethysmogram templates 41b from photoplethysmogram waveform CPPG.

During a stage S62b of flowchart 60b, recorder 40b identifies a dominant photoplethysmogram template 41b for each interval of consecutive intervals (overlapping or non-overlapping) of photoplethysmogram waveform CPPG. For example, as shown in stage S62a, photoplethysmogram waveform CPPG is segmented into six (6) intervals 72a-72f whereby a dominant morphology within each interval 72 of photoplethysmogram waveform CPPG is extracted and designated as a dominant photoplethysmogram template 41b.

A dominant morphology of an interval of photoplethysmogram waveform CPPG is the morphology best representative of a photoplethysmogram status of the monitored subject during the interval of photoplethysmogram waveform CPPG for diagnostic monitoring and interpretation purposes.

Examples of a dominant morphology of an interval of photoplethysmogram waveform CPPG include, but are not limited to, a mean morphology or function thereof among the cardiac cycles within the interval of photoplethysmogram waveform CPPG, a median morphology or function thereof among the cardiac cycles of within the interval of photoplethysmogram waveform CPPG, and a mode morphology or function thereof among the cardiac cycles of within the interval of photoplethysmogram waveform CPPG.

At the conclusion of stage S62b, recorder 40 temporally aligns the dominant photoplethysmogram templates 41b as a representation of a dominant photoplethysmogram rhythm DPPGR, which may be communicated to analyzer 50b as dominant photoplethysmogram waveform as shown or as individual dominant photoplethysmogram templates 41b in sequential order.

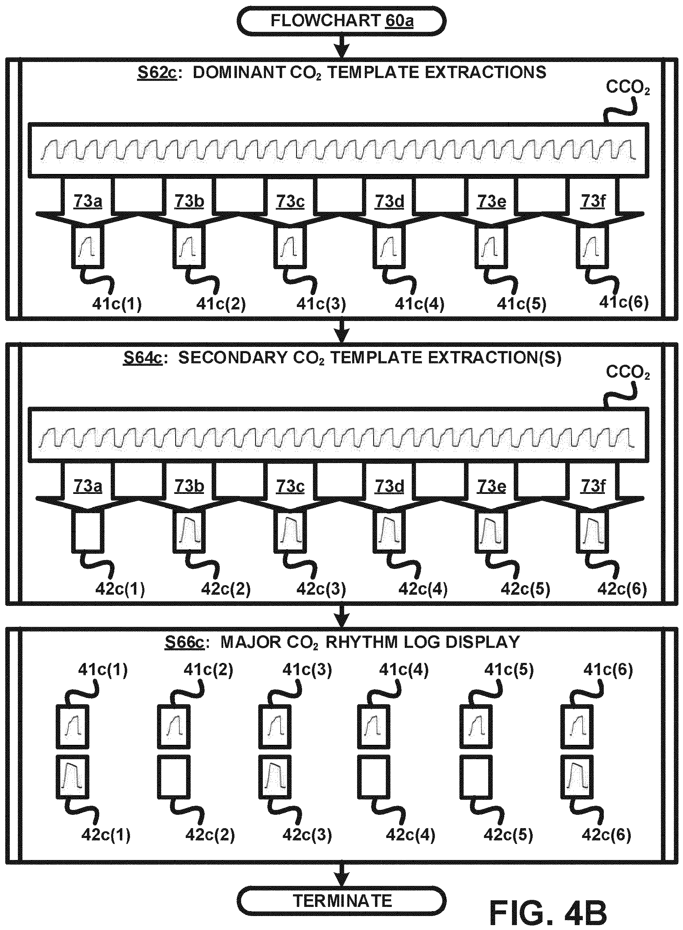

In a third exemplary embodiment of monitor 30 as shown in FIG. 4A, a capnogram waveform summarizing monitor 30c employs a capnogram waveform summarizing recorder 40c and a capnogram waveform summarizing analyzer 50c for implementing a capnogram waveform summarizing method of the present disclosure as represented by a flowchart 60c shown in FIG. 4B.

Recorder 40c records capnogram waveform CCO₂ as received from capnogram waveform interface 20a. Concurrently or subsequently to the recording of capnogram waveform CCO₂, recorder 40c extracts dominant capnogram templates 41b from capnogram waveform CCO₂.

During a stage S62c of flowchart 60c, recorder 40c identifies a dominant capnogram template 41b for each interval of consecutive intervals (overlapping or non-overlapping) of capnogram waveform CCO₂. For example, as shown in stage S62a, capnogram waveform CCO₂ is segmented into six (6) intervals 73a-73f whereby a dominant morphology within each interval 73 of capnogram waveform CCO₂, is extracted and designated as dominant capnogram template 41b.

A dominant morphology of an interval of capnogram waveform CCO₂ is the morphology best representative of a capnogram status of the monitored subject during the interval of capnogram waveform CCO₂ for diagnostic monitoring and interpretation purposes.

Examples of a dominant morphology of an interval of capnogram waveform CCO₂ include, but are not limited to, a mean morphology or function thereof among the respiratory cycles within the interval of capnogram waveform CCO₂, a median morphology or function thereof among the respiratory cycles of within the interval of capnogram waveform CCO₂, and a mode morphology or function thereof among the respiratory cycles of within the interval of capnogram waveform CCO₂.

At the conclusion of stage S62c, recorder 40 temporally aligns the dominant capnogram templates 41c as a representation of a dominant capnogram rhythm DCO₂R, which may be communicated to analyzer 50c as dominant capnogram waveform as shown or as individual dominant capnogram templates 41c in sequential order.

Referring back to FIG. 1B, concurrently or subsequently to the recording of physiological waveform CPW, recorder 40 may extract one or more secondary physiological templates 42 from physiological waveform CPW.

During a stage S64 of flowchart 60, for each interval of physiological waveform CPW, recorder 40 determines a presence or an absence of a secondary major physiological rhythm with physiological waveform CPW whereby a detected presence of a secondary major physiological rhythm within an interval is extracted and designated as a secondary physiological template 42.

An exemplary implementation of stage S64, as shown in FIG. 1B, involves a segmenting of physiological waveform CPW into six (6) intervals 70a-70f whereby a presence of a secondary major physiological rhythm within each interval 70 of physiological waveform CPW is investigated and each identified secondary major physiological rhythm is designated as a secondary physiological template 42. FIG. 2B exemplary illustrates a first interval, a third interval and a sixth interval as having an identified secondary major physiological rhythm.

At the conclusion of stage S64, recorder 40 temporally aligns the secondary physiological templates 41 to as a representation of the secondary major physiological rhythm SMPR of physiological waveform CPW, which may be communicated to analyzer 50 as secondary physiological waveform as shown or as individual secondary physiological templates 42 in sequential order.

In one exemplary embodiment of stage S64 as shown in FIG. 2B, concurrently or subsequently to the recording of electrocardiogram waveform CECG, a stage S64a of flowchart 60a may encompass recorder 40a (FIG. 2A) extracting one or more secondary electrocardiogram templates 42a from electrocardiogram waveform CECG.

During a stage S64a of flowchart 60a, for each interval of electrocardiogram waveform CECG, recorder 40a determines a presence or an absence of secondary major electrocardiogram rhythm with electrocardiogram waveform CECG and embodies any identified secondary major electrocardiogram rhythm within a secondary electrocardiogram template 42a.

An exemplary implementation of stage S64a, as shown in FIG. 2B, involves a segmenting of electrocardiogram waveform CECG into six (6) intervals 71a-71f whereby a presence of a secondary major electrocardiogram rhythm within each interval 71 of electrocardiogram waveform CECG is investigated and each identified secondary major electrocardiogram rhythm is designated as a secondary electrocardiogram template 42. FIG. 2B exemplary illustrates a first interval, a third interval and a sixth interval as having an identified secondary major electrocardiogram rhythm.

At the conclusion of stage S64a, recorder 40a temporally aligns the secondary electrocardiogram templates 42a as a representation of the secondary electrocardiogram rhythm SECGR of electrocardiogram waveform CECG, which may be communicated to analyzer 50a (FIG. 2A) as a secondary electrocardiogram waveform as shown or as individual secondary electrocardiogram templates 42a in sequential order.

In a second exemplary embodiment of stage S64 as shown in FIG. 3B, concurrently or subsequently to the recording of photoplethysmogram waveform CPPG, a stage S64b of flowchart 60b may encompass recorder 40b (FIG. 3A) extracting one or more secondary photoplethysmogram templates 42b from photoplethysmogram waveform CPPG.

During a stage S64b of flowchart 60b, for each interval of photoplethysmogram waveform CPPG, recorder 40b determines a presence or an absence of a secondary major photoplethysmogram rhythm with photoplethysmogram waveform CPPG and embodies any identified secondary major photoplethysmogram rhythm within a photoplethysmogram template 42b.

An exemplary implementation of stage S64b, as shown in FIG. 3B, involves a segmenting of photoplethysmogram waveform CPPG into six (6) intervals 72a-72f whereby a presence of a secondary major photoplethysmogram rhythm within each interval 72 of photoplethysmogram waveform CPPG is investigated and each identified secondary major photoplethysmogram rhythm is designated as a secondary photoplethysmogram template 42. FIG. 3B exemplary illustrates a first interval, a third interval and a sixth interval as having an identified secondary major photoplethysmogram rhythm.

At the conclusion of stage S64a, recorder 40b temporally aligns the secondary photoplethysmogram templates 42b as a representation of the secondary photoplethysmogram rhythm SPPGR of photoplethysmogram waveform CPPG, which may be communicated to analyzer 50b (FIG. 3A) as a secondary photoplethysmogram waveform as shown or as individual secondary photoplethysmogram templates 42b in sequential order.

In a third exemplary embodiment of stage S64 as shown in FIG. 4B, concurrently or subsequently to the recording of capnogram waveform CCO₂, a stage S64c of flowchart 60c may encompass recorder 40c (FIG. 4A) extracting one or more secondary capnogram templates 42c from capnogram waveform CCO₂.

During a stage S64c of flowchart 60c, for each interval of capnogram waveform CCO₂, recorder 40c determines a presence or an absence of a secondary major capnogram rhythm with capnogram waveform CCO₂ and embodies a secondary major capnogram rhythm within a capnogram template 42c.

An exemplary implementation of stage S64c, as shown in FIG. 4B, involves a segmenting of capnogram waveform CCO₂ into six (6) intervals 73a-73f
whereby a presence of a secondary major capnogram rhythm within each interval 73 of capnogram waveform CCO₂ is investigated and each identified secondary major capnogram rhythm is designated as a secondary capnogram template 43. FIG. 4B exemplary illustrates a first interval, a third interval and a sixth interval as having an identified secondary major capnogram rhythm.

At the conclusion of stage S64c, recorder 40c temporally aligns the secondary capnogram templates 42c as a representation of the secondary capnogram rhythm SCO₂R of capnogram waveform CCO₂, which may be communicated to analyzer 50c (FIG. 4A) as a secondary capnogram waveform as shown or as individual secondary capnogram templates 42c in sequential order.

Referring back to FIG. 1B, upon receipt of dominant physiological templates 41of physiological waveform CPW and upon the receipt of secondary physiological template(s) 42 of physiological waveform CPW, if implemented, physiological waveform summarizing analyzer 50 facilitates an analysis of the templates via a user interface as known in the art enabling a user to delineate and measure physiological parameter(s) and/or variations thereof within templates, and/or a via an autonomous delineation and measurement by analyzer 50 of physiological parameter(s) and variations thereof within the templates.

During a stage S66 of flowchart 60, analyzer 50 may display a dominating major physiological rhythm log including an integrated or a segregated display of a subset or the entire set of dominant physiological templates 41 extracted from physiological waveform CPW during stage S62, and may further include an individual, an integrated or a segregated display of secondary physiological template(s) 42 extracted from physiological waveform CPW during stage S64, if implemented.

FIG. 1B illustrates a segregated display of the set of dominant physiological templates 41 extracted from physiological waveform CPW during the exemplary implementation of stage S62 as shown in FIG. 1B, and a segregated display of the secondary physiological templates 42 extracted from physiological waveform CPW during the exemplary implementation of stage S64 as shown in FIG. 1B. Templates 41 and 42 of the same interval of physiological waveform CPW may be vertically aligned as shown or horizontally aligned.

The display of templates 41, 42 as shown in FIG. 1B may include a graphical user interface (not shown) as known in the art of the present disclosure or hereinafter conceived, enabling a user to delineate and measure physiological parameter(s) and/or variations thereof of the templates 41, 42.

Alternatively or concurrently, the display of templates 41, 42 as shown in FIG. 1B may include an autonomous delineation and measurements by analyzer 50 (not shown) of physiological parameter(s) and/or variations thereof of the templates 41, 42.

In one exemplary embodiment of stage S66 of flowchart 60 as shown in FIG. 2B, during as stage S66a, analyzer 50a may display a dominating major physiological rhythm log including an integrated or a segregated display of a subset or the entire set of dominant electrocardiogram templates 41a extracted from electrocardiogram waveform CECG during stage S62a, and may further include an individual, an integrated or a segregated display of secondary electrocardiogram template(s) 42a extracted from electrocardiogram waveform CECG during stage S64a, if implemented.

FIG. 2B illustrates a segregated display of the set of dominant electrocardiogram templates 41a extracted from electrocardiogram waveform CECG during the exemplary implementation of stage S62a as shown in FIG. 2B, and a segregated display of the secondary electrocardiogram templates 42a extracted from electrocardiogram waveform CECG during the exemplary implementation of stage S64a as shown in FIG. 2B. Templates 41a and 42a of the interval of electrocardiogram waveform CECG may be vertically aligned as shown or horizontally aligned.

The display of templates 41a, 42a as shown in FIG. 2B may include a graphical user interface (not shown) as known in the art of the present disclosure or hereinafter conceived, enabling a user to delineate and measure electrocardiogram parameter(s) and/or variations thereof of the templates 41a, 42a.

In a second exemplary embodiment of stage S66 of flowchart 60 as shown in FIG. 3B, during a stage S66b, analyzer 50b may display a dominating major physiological rhythm log including an integrated or a segregated display of a subset or the entire set of dominant photoplethysmogram templates 41b extracted from photoplethysmogram waveform CPPG during stage S62b, and may further include an individual, an integrated or a segregated display of secondary photoplethysmogram template(s) 42b extracted from photoplethysmogram waveform CPPG during stage S64b, if implemented.

FIG. 3B illustrates a segregated display of the set of dominant photoplethysmogram templates 41b extracted from photoplethysmogram waveform CPPG during the exemplary implementation of stage S62b as shown in FIG. 3B, and a segregated display of the secondary photoplethysmogram templates 42b extracted from photoplethysmogram waveform CPPG during the exemplary implementation of stage S64b as shown in FIG. 3B. Templates 41b and 42b of the same interval of photoplethysmogram waveform CPPG may be vertically aligned as shown or horizontally aligned.

The display of templates 41b, 42b as shown in FIG. 3B may include a graphical user interface (not shown) as known in the art of the present disclosure or hereinafter conceived, enabling a user to delineate and measure photoplethysmogram parameter(s) and/or variations thereof of the templates 41b, 42b.

In a third exemplary embodiment of stage S66 of flowchart 60 as shown in FIG. 4B, during a stage S66c, analyzer 50c may display a dominating major physiological rhythm log including an integrated or a segregated display of a subset or the entire set of dominant capnogram templates 41b extracted from capnogram waveform CCO₂ during stage S62c, and may further include an individual, an integrated or a segregated display of secondary capnogram template(s) 42b extracted from capnogram waveform CCO₂ during stage S64c, if implemented.

FIG. 4B illustrates a segregated display of the set of dominant capnogram templates 41c extracted from capnogram waveform CCO₂ during the exemplary implementation of stage S62c as shown in FIG. 4B, and a segregated display of the secondary capnogram templates 42bc extracted from capnogram waveform CCO₂ during the exemplary implementation of stage S64c as shown in FIG. 4B. Templates 41c and 42c of the same interval of capnogram waveform CCO₂ may be vertically aligned as shown or horizontally aligned.

The display of templates 41c, 42c as shown in FIG. 4B may include a graphical user interface (not shown) as known in the art of the present disclosure or hereinafter conceived, enabling a user to delineate and measure capnogram parameter(s) and/or variations thereof of the templates 41c, 42c.

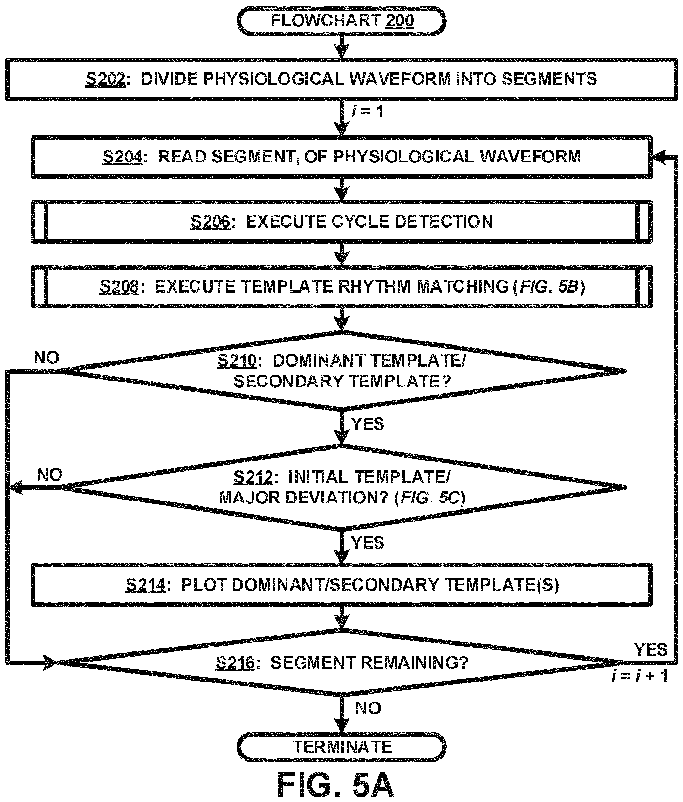

To facilitate an understanding of the present disclosure, the following description of FIGS. 5A-5C teaches exemplary embodiments of physiological waveform summarizing method of FIG. 1A in accordance with the present disclosure. From the description of FIGS. 5A-5C, those having ordinary skill in the art of the present disclosure will appreciate how to apply the present disclosure to make and use additional embodiments of physiological waveform summarizing methods in accordance with the present disclosure.

Referring to FIG. 5A, a flowchart 200 represents an electrocardiogram summarizing method in accordance with the present disclosure.

A stage S202 of flowchart 200 encompasses a monitor of the present disclosure dividing a physiological waveform into an X number of overlapping or non-overlapping segments, X ≥ 2.

For example, a thirty (30) minute physiological waveform may be divided into thirty (30) one-minute non-overlapping segments.

A stage S204 of flowchart 200 encompasses the monitor of the present disclosure reading of a first segment in a manner to facilitate a cycle detection by the monitor of the present disclosure during a stage S206 of flowchart 200 and a template rhythm matching by the monitor of the present disclosure during a stage S208 of flowchart 200.

Still referring to FIG. 5A, the cycle detection of stage S206 identifies each cycle, if any, within the first segment of the physiological waveform as known in the art of the present disclosure or hereinafter conceived.

In one exemplary embodiment of stage S206 for an electrocardiogram, the cycle detection identifies each cardiac cycle (pulse), if any, within the first segment of the electrocardiogram as known in the art of the present disclosure or hereinafter conceived.

In a second exemplary embodiment of stage S206 for a photoplethysmogram, the cycle detection identifies each cardiac cycle (pulse), if any, within the first segment of the photoplethysmogram as known in the art of the present disclosure or hereinafter conceived.

In a third exemplary embodiment of stage S206 for capnogram, the cycle detection identifies each respiratory cycle (breath), in any, within the first segment of the capnogram as known in the art of the present disclosure or hereinafter conceived.

Still referring to FIG. 5A, the template rhythm matching of stage S208 classifies each identified cycle within the first segment of the physiological waveform into one of a plurality of matching rhythm groups to facilitate an extraction of a dominant physiological template from the first segment of the physiological waveform during a stage S210 of flowchart 200 and to facilitate an extraction of a secondary physiological template, if any, from the first segment of the physiological waveform during stage S210 of flowchart 200.

In practice of stage S208, supervised learning model(s) and/or unsupervised learning model(s) may serve as a basis for functionally mapping each identified cycle to one of a plurality of matching rhythm groups.

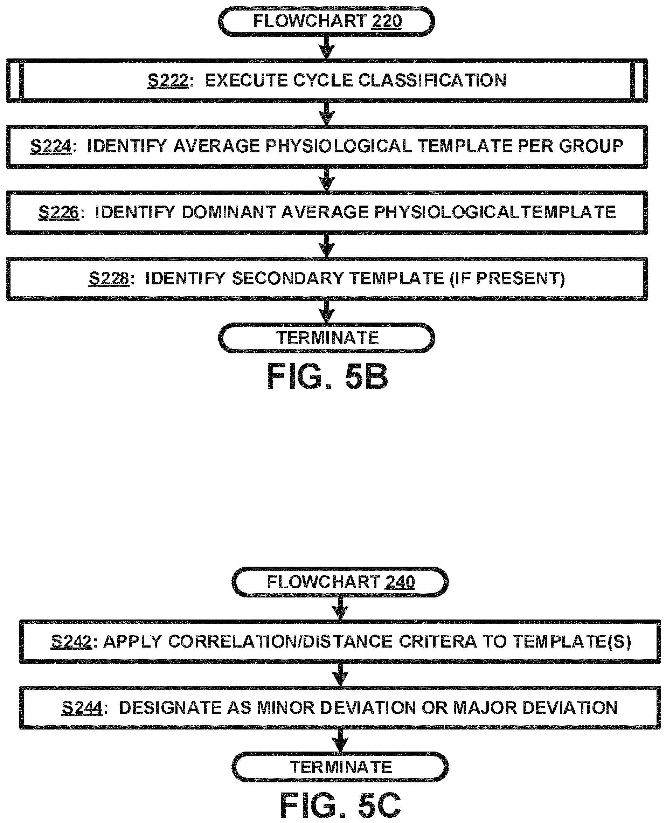

In one exemplary embodiment of stage S208 as shown in FIG. 5B, a flowchart 220 represents an exemplary template rhythm matching method of the present disclosure.

Referring to FIG. 5B, a stage S222 of flowchart 220 encompasses a monitor of the present disclosure inputting the first segment into neural network classifier as known in the art of the present disclosure or hereinafter conceived to thereby functionally cluster or correlate each cycle to one of a rhythm matching group. Examples of the neural network classifier includes, but are not limited to, a linear regression classifier, a decision tree classifier, a random forest classifier, a naive bayes classifier, a nearest neighbor classifier and an artificial intelligence/deep classifier.

In practice, a supervised neural network classifier will be trained on designated rhythms of the physiological waveform. For example, a supervised neural network classifier may be trained on designated rhythms of an electrocardiogram including, but not limited to, a normal rhythm, a Premature Atrial Complex (PAC), a Premature Ventricular Complex (PVC - bigeminy or trigeminy rhythms), an Atrial Fibrillation (AFib), an Atrial Flutter (AFlutter), and a Ventricular Tachycardia (VT).

Alternatively in practice, an unsupervised neural network classifier will be designed to determine and group related rhythms of the physiological waveform. For example, a unsupervised neural network classifier may be designed to determine and group related rhythms of an electrocardiogram including, but not limited to, a normal rhythm, a Premature Atrial Complex (PAC), a Premature Ventricular Complex (PVC - bigeminy or trigeminy rhythms), an Atrial Fibrillation (AFib), an Atrial Flutter (AFlutter), and a Ventricular Tachycardia (VT).

Still referring to FIG. 5B, a stage S224 of flowchart 220 encompasses the monitor of the present disclosure identifying an average physiological template for each rhythm group, a stage S226 of flowchart 220 encompasses the monitor of the present disclosure extracting dominant physiological template of the first segment as the average physiological template of the rhythm group having a maximum number of cycles, and a stage S228 of flowchart 220 encompasses the monitor of the present disclosure identifying a secondary physiological template of the first segment (if present) from the rhythm group having a second highest number of cycles, particularly above a threshold delineating major rhythms from noise and artefacts.

Referring back to FIG. 5C, upon completion of stages S206 and S208, a stage S210 of flowchart 200 encompasses the monitor of the present disclosure ascertaining if a dominant physiological template and/or a secondary physiological template of the first segment was(were) identified during stage S208.

If the first segment of the physiological waveform did not include any cycles (e.g., a pulseless segment of an electrocardiogram or a photoplethysmogram, or a breath-less segment of a capnogram), then neither a dominant physiological template nor a secondary physiological template of the first segment would have been identified during stage S208 and the monitor of the present disclosure would proceed to a stage S216 of the flowchart 200 to ascertain if there are any remaining segments. If there are remaining segments, then the monitor of the present disclosure proceeds to iterate stages S204-S210 for the next segment. Otherwise, flowchart 200 is terminated.

If the first segment of the physiological waveform included one or more cycles (e.g., a pulsing segment of an electrocardiogram or a photoplethysmogram, or a breathing segment of a capnogram), then a dominant physiological template of the first segment and if present, a secondary physiological template of the first segment would have been identified during stage S208 and the monitor of the present disclosure would proceed to a stage S212 of flowchart 200 to ascertain if the identified template(s) of stage S208 are within the first segment.

If the identified templates of stage S208 are within the first segment, then the monitor of the present disclosure proceeds to a stage S214 of flowchart 200 to plot the identified template(s) within a major physiological rhythm log.

If the identified template(s) of stage S208 are not within the first segment, then the monitor of the present disclosure proceeds to stage S214 to plot an identified dominant physiological template within a major physiological rhythm log if the dominant physiological template is a major deviation from a previously plotted dominant physiological template within the major physiological rhythm log and/or to plot an identified secondary physiological template within the major physiological rhythm log if the secondary physiological template is a major deviation from a previously plotted secondary physiological template within the major physiological rhythm log.

In practice of stage S212, a flowchart 240 of FIG. 5C represents a physiological template selection method of the present disclosure. Referring to FIG. 5C, for an identified dominant physiological template of stage S208, a stage S242 of flowchart 240 encompasses a monitor of the present disclosure applying correlation criteria or distance criteria to the previously plotted dominant physiological template and the identified dominant physiological template to derive a deviation between the templates, and a stage S244 of flowchart 240 encompasses the monitor of the present disclosure designating the deviation as a minor deviation if the deviation is below a predefined threshold, or as a major deviation is a deviation between templates exceeds the predefined threshold.

Referring back to FIG. 5A, upon completion of stage S214 or if a major template deviation was not determined during stage S212, then the monitor of the present disclosure proceeds to stage S216 to ascertain if there are any remaining segments. If there are remaining segments, then the monitor of the present disclosure proceeds to iterate stages S204-S210 for the next segment. Otherwise, flowchart 200 is terminated.

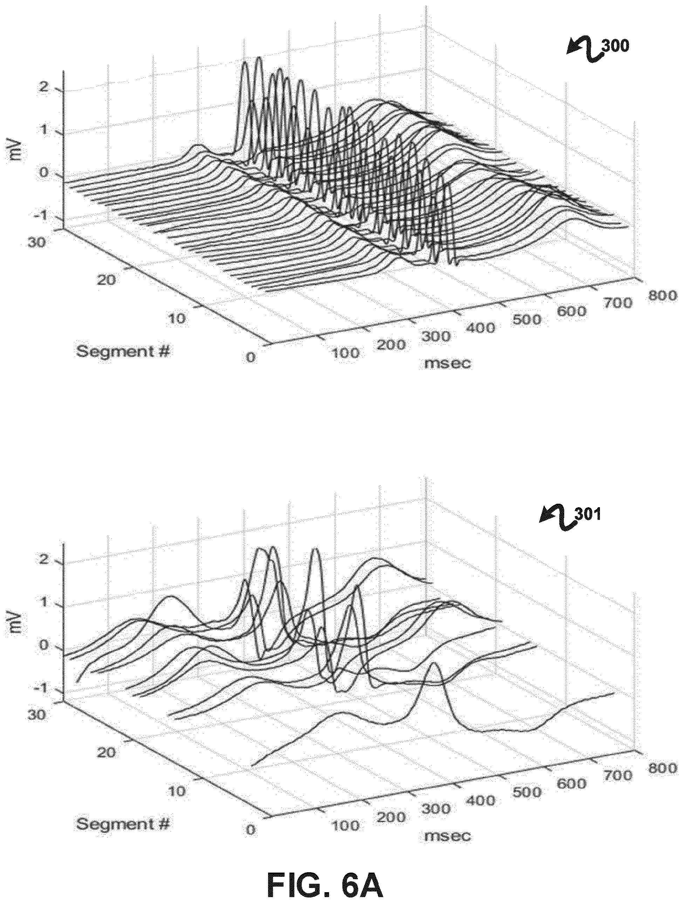

FIGS. 6A-6C illustrate an example implementation of flowchart 200 (FIG. 5A), flowchart 220 (FIG. 5B) and flowchart 240 (FIG. 5C) in the context of an electrocardiogram having annotated episodes of normal and bigeminy rhythms. Specifically, a 30-minute record of electrocardiogram is split into 1-minute non-overlapping segments and the flowcharts are executed on each segment. More particularly, the cycle-detection algorithm identified the cardiac cycles (pulses) in each segment. The template rhythm matching algorithm based on a neural network classifier matched each cardiac cycle to a group (cluster)(correlation), found the average ECG templates in each group, and identified the dominant ECG template and (if present) secondary ECG template based on the number of cardiac cycles in each group. Other than the normal rhythm which is shown in dominant ECG templates, a secondary ECG template is present in some segments which is associated with PVC beats in the bigeminy rhythm.

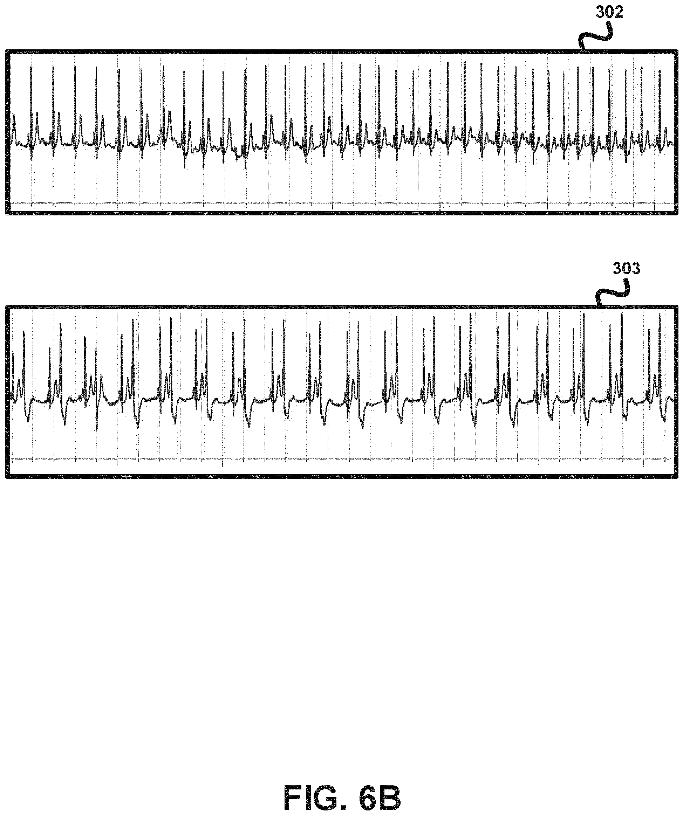

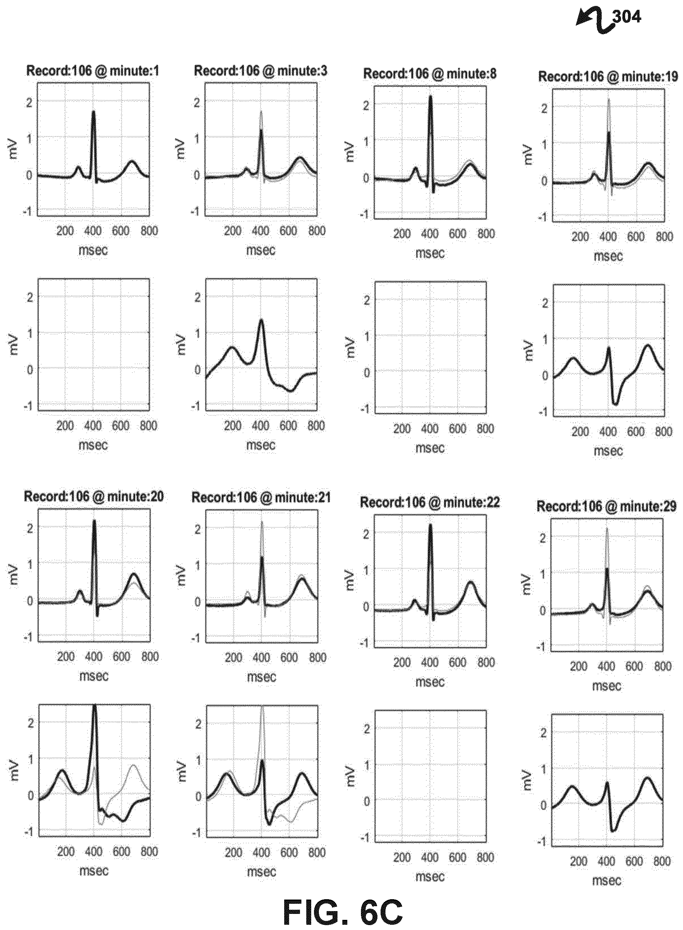

More particularly, FIG. 6A shows a plotting 300 of the dominant ECG templates and a plotting 301 of the secondary ECG templates over time for all thirty (30) 1-minute segments. FIG. 6B shows example intervals of a record bearing normal rhythm 302 and a bigeminy rhythm 303. FIG. 6C shows a major ECG rhythm log 304 of average ECG templates at 1-minute segments wherever a major change in a dominant ECG template or a secondary ECG template is detected whereby the dominant ECG templates show the average normal beats and the secondary ECG templates are PVC beats related to the bigeminy rhythm.

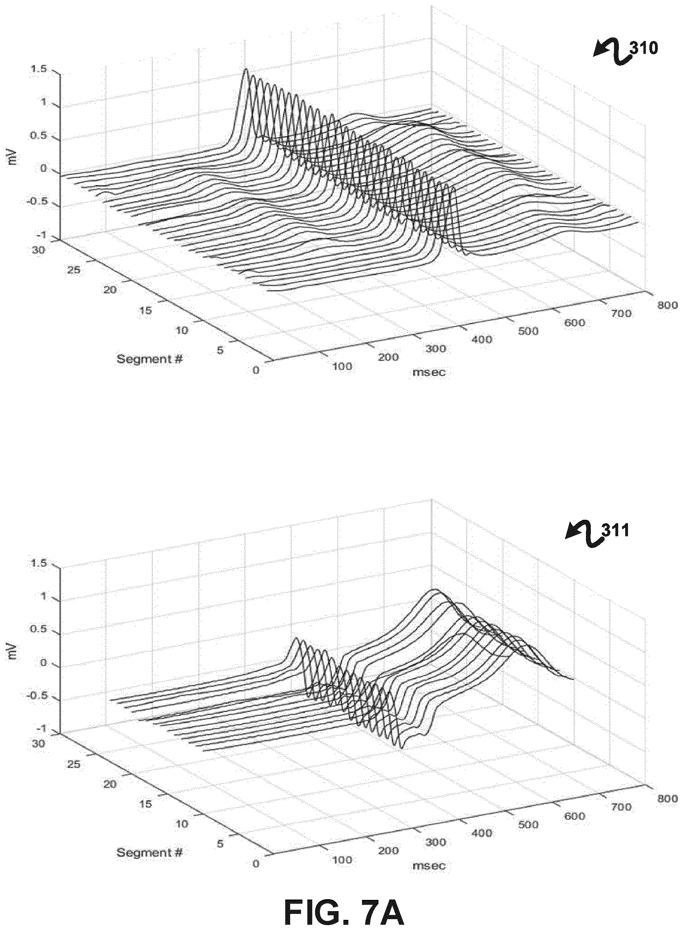

FIGS. 7A-7C illustrate an example implementation of flowchart 200 (FIG. 5A), flowchart 220 (FIG. 5B) and flowchart 240 (FIG. 5C) in the context of an electrocardiogram having annotated episodes of AFib and trigeminy rhythms. Specifically, a 30-minute record of electrocardiogram is split into 1-minute non-overlapping segments and the flowcharts are executed on each segment. More particularly, the cycle-detection algorithm identified the cardiac cycles (pulses) in each segment. The template rhythm matching algorithm based on a neural network classifier matched each cardiac cycle to a group (cluster)(correlation), found the average ECG templates in each group, and identified the dominant ECG template and (if present) secondary ECG template based on the number of cardiac cycles in each group. Other than the dominant ECG templates in all segments which relate to either AFib or normal rhythms, a secondary ECG template is present in some segments associated with PVC beats in trigeminy rhythm.

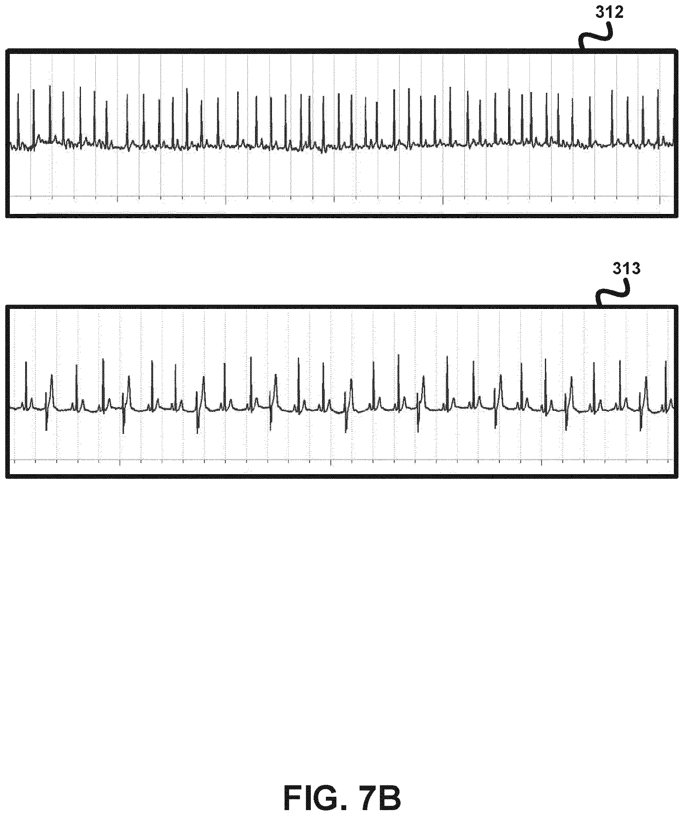

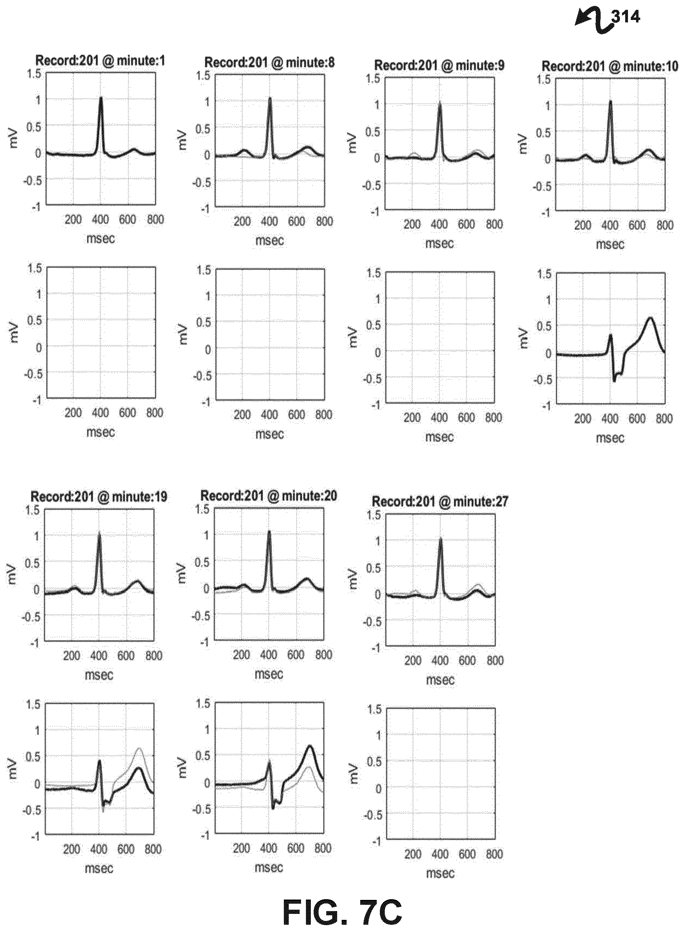

More particularly, FIG. 7A shows a plotting 310 of the dominant ECG templates and a plotting 311 of the secondary ECG templates over time for all thirty (30) 1-minute segments. FIG. 7B shows example intervals of a record bearing AFib rhythm 312 and a trigeminy rhythm 313. FIG. 7C shows a major ECG rhythm log 314 of average ECG templates at 1-minute segments wherever a major change in a dominant ECG template or a secondary ECG template is detected whereby the dominant ECG templates show pulses lacking p-wave are related to AFib rhythm and the secondary ECG templates show PVC pulses in trigeminy rhythm.

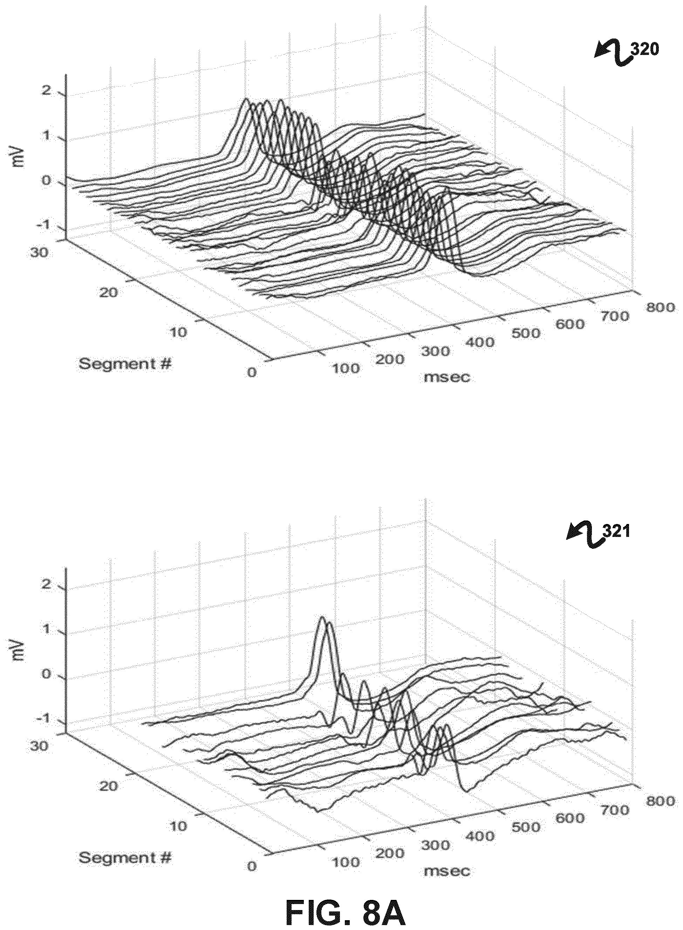

FIGS. 8A-8C illustrate an example implementation of flowchart 200 (FIG. 5A), flowchart 220 (FIG. 5B) and flowchart 240 (FIG. 5C) in the context of an electrocardiogram having annotated episodes of AFib and AFlutter. Specifically, a 30-minute record of electrocardiogram is split into 1-minute non-overlapping segments and the flowcharts are executed on each segment. More particularly, the cycle-detection algorithm identified the cardiac cycles (pulses) in each segment. The template rhythm matching algorithm based on a neural network classifier matched each cardiac cycle to a group (cluster)(correlation), found the average ECG templates in each group, and identified the dominant ECG template and (if present) secondary ECG template based on the number of cardiac cycles in each group. The dominant ECG templates mostly relate to AFib rhythm, while secondary ECG templates mostly present the atrial flutter rhythm.

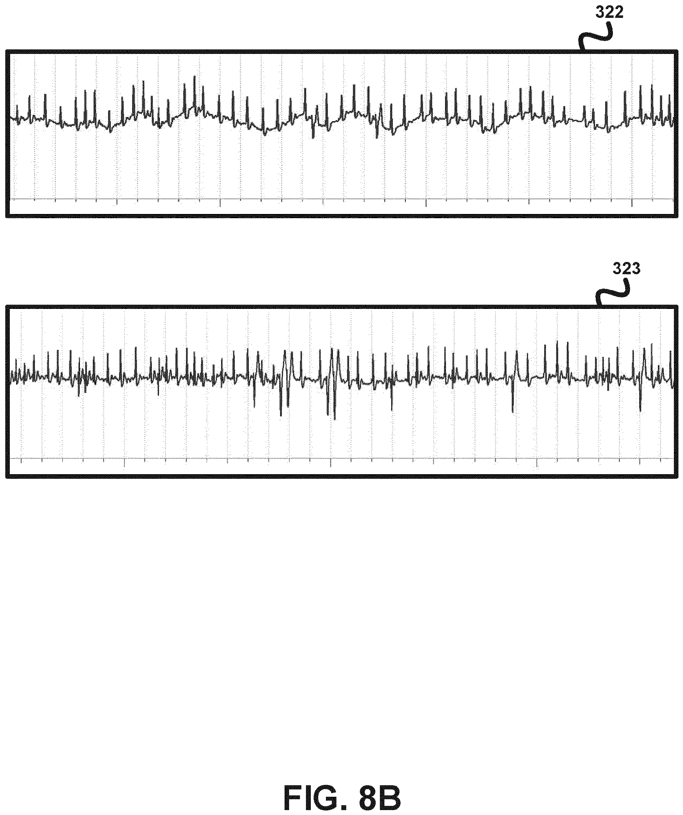

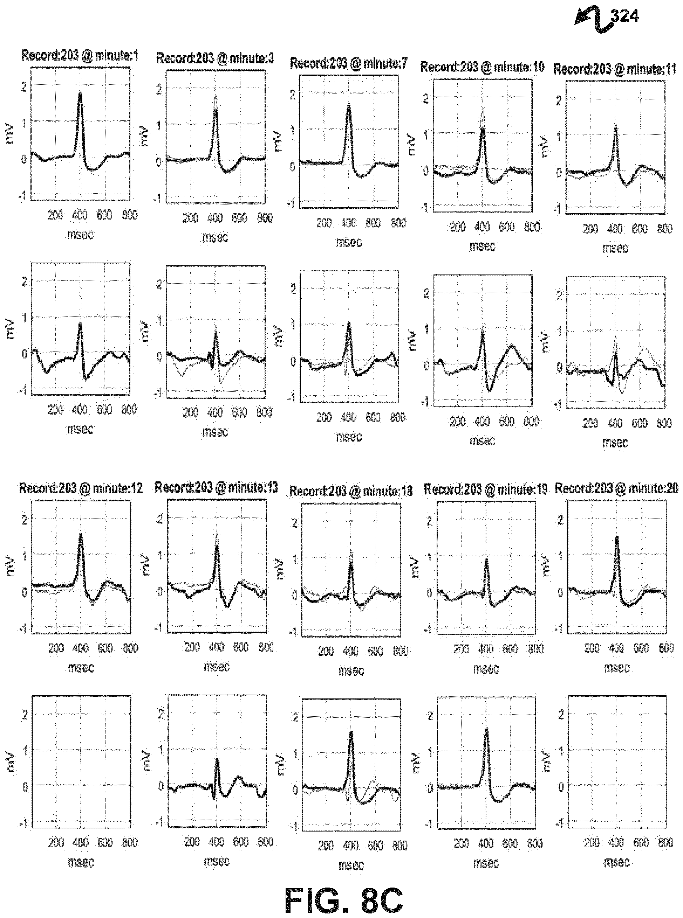

More particularly, FIG. 8A shows a plotting 320 of the dominant ECG templates and a plotting 321 of the secondary ECG templates over time for all thirty (30) 1-minute segments. FIG. 8B shows example intervals of a record bearing AFib rhythm 322 and a AFlutter rhythm 323. FIG. 8C shows a major ECG rhythm log 324 of average ECG templates at 1-minute segments wherever a major change in a dominant ECG template or a secondary ECG template is detected whereby the dominant ECG templates are mostly related to AFib rhythm and the secondary ECG templates mostly show AFlutter rhythm.

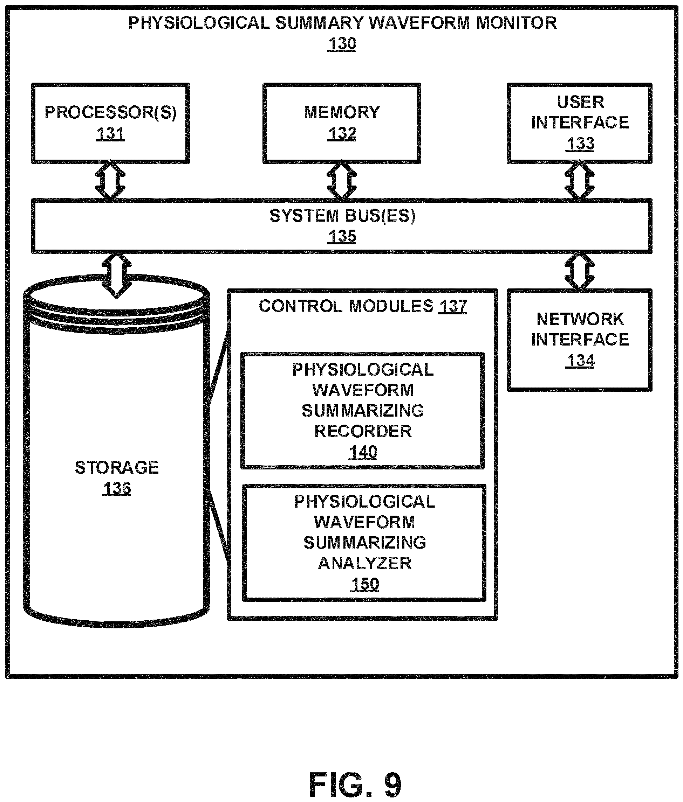

To facilitate an understanding of the present disclosure, the following description of FIG. 9 teaches exemplary embodiments of physiological waveform summarizing monitor of FIG. 1A in accordance with the present disclosure. From the description of FIG. 9, those having ordinary skill in the art of the present disclosure will appreciate how to apply the present disclosure to make and use additional embodiments of the physiological waveform summarizing monitor of FIG. 1A in accordance with the present disclosure.

Referring to FIG. 9, an exemplary embodiment 130 of physiological waveform summarizing monitor 30 (FIG. 1) includes one or more processor(s) 131, memory 132, a user interface 133, a network interface 134, and a storage 136 interconnected via one or more system buses 135.

Each processor 131 may be any hardware device, as known in the art of the present disclosure or hereinafter conceived, capable of executing instructions stored in memory 132 or storage or otherwise processing data. In a non-limiting example, the processor(s) 131 may include a microprocessor, field programmable gate array (FPGA), application-specific integrated circuit (ASIC), or other similar devices.

The memory 132 may include various memories, as known in the art of the present disclosure or hereinafter conceived, including, but not limited to, L1, L2, or L3 cache or system memory. In a non-limiting example, the memory 132 may include static random access memory (SRAM), dynamic RAM (DRAM), flash memory, read only memory (ROM), or other similar memory devices.

The user interface 133 may include one or more devices, as known in the art of the present disclosure or hereinafter conceived, for enabling communication with a user such as an administrator. In a non-limiting example, the user interface may include a command line interface or graphical user interface that may be presented to a remote terminal via the network interface 134.

The network interface 134 may include one or more devices, as known in the art of the present disclosure or hereinafter conceived, for enabling communication with ventilation system 220, pharmacological source(s) 250, physiological sources(s) 260, psychological source(s) 270 and an electronic medical records system 210. In a non-limiting example, the network interface 134 may include a network interface card (NIC) configured to communicate according to the Ethernet protocol. Additionally, the network interface 134 may implement a TCP/IP stack for communication according to the TCP/IP protocols. Various alternative or additional hardware or configurations for the network interface 134 will be apparent.

The storage 136 may include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 136 may store instructions for execution by the processor(s) 131 or data upon with the processor(s) 131 may operate. For example, the storage 136 may store a base operating system for controlling various basic operations of the hardware. The storage 136 also stores application modules physiological waveform summarizing recorder 140 and physiological waveform summarizing analyzer 150 in the form of executable software/firmware for implementing the various functions of physiological waveform summarizing recorder 40 (FIG. 1) and physiological waveform summarizing analyzer 50 (FIG. 1) as previously described in the present disclosure.

In practice, physiological waveform summarizing monitor 130 may be a stand-alone monitor including a display, user interface, etc., as known in the art of the present disclosure or hereinafter conceived, or may be incorporated/integrated as a controller into devices and systems for acquiring, monitoring and/or interpreting physiological waveforms as known in the art of the present disclosure or hereinafter conceived. Examples of such devices and systems include, but are not limited to, defibrillators, pacemakers, ECG monitors, PPG monitors, CO₂ monitors, Holter monitors and stress test systems.

Referring to FIGS. 1-9, those having ordinary skill in the art of the present disclosure will appreciate numerous benefits of the present disclosure including, but not limited to, a summarizing of a physiological waveform to facilitate a more accurate and reliable diagnostic monitoring and interpretation of a physiological waveform.

Further, as one having ordinary skill in the art will appreciate in view of the teachings provided herein, structures, elements, components, etc. described in the present disclosure / specification and/or depicted in the Figures may be implemented in various combinations of hardware and software, and provide functions which may be combined in a single element or multiple elements. For example, the functions of the various structures, elements, components, etc. shown/illustrated/depicted in the Figures can be provided through the use of dedicated hardware as well as hardware capable of executing software in association with appropriate software for added functionality. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar function, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the various and numerous inventions of the present disclosure (which embodiments are intended to be illustrative and not limiting), it is noted that modifications and variations can be made by persons skilled in the art in light of the teachings provided herein, including the Figures. It is therefore to be understood that changes can be made in/to the preferred and exemplary embodiments of the present disclosure which are within the scope of the embodiments disclosed herein. Moreover, it is contemplated that corresponding and/or related systems incorporating and/or implementing the device/system or such as may be used/implemented in/with a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. A machine-implemented physiological waveform summarization method for summarizing a physiological waveform (CPW) having a dominating major physiological rhythm (DMPR), the method comprising:
receiving a physiological waveform (CPW); and
extracting a set of dominant physiological templates (41) from the physiological waveform (CPW),
wherein the set of dominant physiological templates (41) represent the dominating major physiological rhythm (DMPR) of the physiological waveform (CPW) temporally spanning over consecutive intervals of the physiological waveform (CPW), and
wherein each dominant physiological template (41) is derived from a different interval of the consecutive intervals of the physiological waveform (CPW).

2. The physiological waveform summarization method of claim 1, further comprising:
providing a diagnostic major physiological rhythm log of the physiological waveform (CPW) including a diagnostic plotting of the set of dominant physiological templates (41).

3. The physiological waveform summarizing method of claim 1 or 2, comprising:
reading an interval of the physiological waveform (CPW), wherein the read interval includes a plurality of physiological cycles; and
identifying a dominant morphology among the plurality of physiological cycles within the read interval of the physiological waveform (CPW).

4. The physiological waveform summarizing method of any of the preceding claims, comprising:
reading an interval of the physiological waveform (CPW), wherein the read interval includes a plurality of physiological cycles;
matching each physiological cycle of the read interval of the physiological waveform (CPW) into one cluster among a plurality of different clusters;
deriving an average physiological template within each cluster; and
identifying the average physiological template having a dominant morphology among the plurality of average physiological templates.

5. The physiological waveform summarization method of any of the preceding claims,
wherein the physiological waveform (CPW) includes at least one secondary major physiological rhythm (SMPR); and
wherein the physiological waveform summarization method further comprises:
extracting at least one secondary physiological template (42) from the physiological waveform (CPW),
wherein the at least one secondary physiological template (42) represents the at least one secondary major physiological rhythm (SMPR) of the physiological waveform (CPW) temporally present within at least one interval of the physiological waveform (CPW).

6. The physiological waveform summarization method of claim 5, further comprising:
providing a diagnostic major physiological rhythm log of the physiological waveform (CPW) including a diagnostic plotting of the set of dominant physiological templates (41) and the at least one secondary physiological template (42).

7. The physiological waveform summarizing system of claim 5, further comprising:
reading an interval of the physiological waveform (CPW); and
identifying a presence or an absence of an abnormality of the physiological waveform (CPW) within the read interval of the physiological waveform (CPW).

8. The physiological waveform summarization method of claim 1, wherein extracting the set of dominant physiological templates (41) from the physiological waveform (CPW) includes:
reading an interval of the physiological waveform (CPW), wherein the read interval includes a plurality of physiological cycles;
matching each physiological cycle of the read interval of the physiological waveform (CPW) into one cluster among a plurality of different clusters;
deriving an average physiological template within each cluster; and
identifying the average physiological template having the dominant morphology among the plurality of average physiological templates.

9. The physiological waveform summarizing method of any of the preceding claims, wherein the physiological waveform (CPW) is one of an electrocardiogram waveform, a photoplethysmogram waveform and a capnogram waveform.

10. A computer program product comprising instructions for a processor to carry out the method of any of the preceding claims.

11. A physiological waveform summarizing monitor (30) configured to carry out the method of any of the preceding claims 1-9.

12. A physiological waveform summarizing system comprising:
a physiological waveform interface (20); and
a physiological waveform summarizing monitor (30) as claimed in claim 11.
